# EUROPÄISCHE PATENTSCHRIFT

(11) **EP 3 197 417 B1**
(45) Veröffentlichungstag und Bekanntmachung des Hinweises auf die Patenterteilung: **19.08.2020**
(21) Anmeldenummer: 15775136.3
(22) Anmeldetag: 25.09.2015
(51) Int. Cl.: A61J 1/10, G09F 3/04

(54) **VERFAHREN ZUM INDIVIDUELLEN KENNZEICHNEN EINES UNTER FOLIE ODER IN EINEM BEUTEL VORLIEGENDEN MEDIZINISCHEN PRODUKTS UND GEKENNZEICHNETES PRODUKT**
METHOD FOR INDIVIDUALLY IDENTIFYING A MEDICAL PRODUCT LOCATED UNDER A FILM OR IN A POUCH, AND IDENTIFIED PRODUCT
PROCÉDÉ DE CARACTÉRISATION INDIVIDUELLE D'UN PRODUIT MÉDICAL CONDITIONNÉ SOUS UN FILM OU DANS UN SAC, ET PRODUIT AINSI CARACTÉRISÉ

(30) Priorität: 26.09.2014 DE 102014113959
(43) Veröffentlichungstag der Anmeldung: 02.08.2017
(73) Patentinhaber: Fresenius Medical Care Deutschland GmbH, 61352 Bad Homburg (DE)
(72) Erfinder: HÖRMANN, Jörn, 66386 St. Ingbert (DE); MEISINGER, Sebastian, 66646 Marpingen (DE)
(74) Vertreter: Bobbert & Partner Patentanwälte PartmbB
(86) Internationale Anmeldenummer: PCT/EP2015/072152
(87) Internationale Veröffentlichungsnummer: WO 2016/046388

(56) Entgegenhaltungen:
- EP-A2- 2 509 057
- DE-A1- 1 673 107
- DE-A1- 1 946 761
- US-A1- 2003 072 676
- US-A1- 2004 172 007

## Beschreibung

Die vorliegende Erfindung betrifft ein Verfahren gemäß Anspruch 1 zum Kennzeichnen eines in oder unter einer Folie oder in einem Beutel vorliegenden medizinischen Produkts. Zudem betrifft sie ein erfindungsgemäß gekennzeichnetes medizinisches Produkt gemäß Anspruch 3.

Medizinische Produkte wie medizinische Lösungen oder Dialysierfluide, die oftmals in Folien oder Beuteln verpackt sind, werden, insbesondere aufgrund gesetzlicher Vorgaben, gekennzeichnet, um beispielsweise nicht bestimmungsgemäße Anwendungen und/oder Produktfälschungen zu verhindern.

Aus der DE 1 673 107 A1 ist ein Apparat zur Analyse einer Flüssigkeit mit zwei Gefäßen bekannt.

Die DE 1 946 761 A1 offenbart einen Behälter aus Kunststoff für Blut und Infusionslösungen.

Aus der US 2003/0072676 A1 sind Radiofrequenzen elektromagnetischer Informationssysteme und Verfahren zu deren Verwendung in der extrakorporalen Blutbehandlung bekannt.

Der US 2004/0172007 A1 sind Behälter zur Bestrahlung von Blutprodukten zu entnehmen.

In der EP 2 509 057 A2 sind selbstklebende Etiketten zum Befestigen an zerbrechlichen oder leicht verformbaren Objekten offenbart.

Eine Aufgabe der vorliegenden Erfindung ist es, ein weiteres Verfahren zum Kennzeichnen eines in oder unter einer Folie oder in einem Beutel vorliegenden medizinischen Produkts anzugeben.

Ferner soll ein medizinisches Produkt angegeben werden, welches geeignet gekennzeichnet in einer Folie oder in einem Beutel vorliegt.

Die erfindungsgemäße Aufgabe wird durch ein Verfahren mit den Merkmalen des Anspruchs 1 gelöst. Zudem wird sie gelöst durch ein medizinisches Produkt mit den Merkmalen des Anspruchs 3. Erfindungsgemäß wird ein Verfahren zum Kennzeichnen einer Folie oder eines Beutels mit einem medizinischen Produkt oder für die Aufnahme eines medizinischen Produkts, vorgeschlagen. Das medizinische Produkt kann beispielsweise eine medizinische Vorrichtung oder ein medizinisches Verbrauchsmaterial sein.

Die Folie oder der Beutel ist vorgesehen, um das medizinische Produkt aufzunehmen, abzudecken oder anderweitig zu verpacken, oder tut dies.

Das Verfahren ,nicht Teil der Erfindung, umfasst das Verbinden (z. B. Verschweißen oder anderes Fügen) eines Folienstreifens, mit, in, an oder auf wenigstens einem Abschnitt der Folie oder des Beutels, vorzugsweise mit einer Außenseite oder Oberfläche letzterer oder mit sich selbst, vorzugsweise derart, dass sich eine formschlüssige Verbindung ergibt.

Der Folienstreifen trägt, insbesondere optisch erfassbare, Angaben zur Kennzeichnung des medizinischen Produkts, oder weist solche Angaben auf.

Das erfindungsgemäße medizinische Produkt liegt innerhalb oder unter einer Folie oder in einem Beutel vor. Dabei ist wenigstens ein Folienstreifen mit sich selbst verbunden. Der Folienstreifen trägt Angaben zur Kennzeichnung des medizinischen Produkts.

Vorteilhafte Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann nicht erkennbar technisch unmöglich ist. Beides ist von der vorliegenden Erfindung umfasst und gilt für alle hierin verwendeten Zahlenworte.

Zur besseren Lesbarkeit ist im Folgenden vornehmlich von einer Folie die Rede. Diese kann Teil eines Beutels sein, etwa dessen Außenschicht. Wo im Folgenden daher eine Folie genannt ist, gilt dies auch für einen Beutel, sofern dies für den Fachmann nicht erkennbar technisch unmöglich ist.

Das Verbinden ist ein Verschweißen.

Das verwendete Fügeverfahren ist ein Verschweißen.

In manchen erfindungsgemäßen, beispielhaften Ausführungsformen ist das medizinische Verbrauchsmaterial ein Arzneimittel oder eine bei der Dialyse verwendete Lösung.

In gewissen erfindungsgemäßen, beispielhaften Ausführungsformen ist das medizinische Produkt eine Blutkassette, ein Blutschlauchset, ein Teil hiervon, oder dergleichen.

In manchen erfindungsgemäßen, beispielhaften Ausführungsformen ist der Beutel ein Lösungsbeutel, also ein Beutel oder eine Beutelhülle, gefüllt mit Lösung. Die Lösung kann beispielsweise wiederum für die Dialyse vorgesehen sein.

Der Folienstreifen kann jede geeignete Form aufweisen. Er kann als Streifen, Fläche, Abschnitt oder dergleichen ausgestaltet sein.

Der Folienstreifen wird nicht mit der Folie verbunden, sondern ausschließlich mit sich selbst. Es wird also ein erster Abschnitt des Folienstreifens mit einem zweiten Abschnitt des Folienstreifens verbunden.

In gewissen erfindungsgemäßen, beispielhaften Ausführungsformen des Verfahrens ist die Folie in wenigstens einem Abschnitt hiervon wenigstens zweilagig ausgestaltet. Dabei ist oder wird wenigstens eine erste Folienlage auf oder mit einer zweiten Folienlage verbunden wie z. B. verschweißt. Wenigstens ein Abschnitt des Folienstreifens ist oder wird zwischen der ersten Folienlage und der zweiten Folienlage angeordnet und wird mit wenigstens der ersten Folienlage und/oder der zweiten Folienlage, beispielsweise mit jeweils den Innenflächen hiervon, verbunden, beispielsweise verschweißt oder verklebt.

In manchen erfindungsgemäßen, beispielhaften Ausführungsformen wird oder ist der Folienstreifen mit wenigstens einer Außenschicht oder Außenfläche der Folie oder der ersten Folienlage verbunden, beispielsweise verschweißt oder verklebt.

In manchen erfindungsgemäßen, beispielhaften Ausführungsformen umfasst das Verfahren das Erzeugen einer Durchgangsöffnung, etwa eines Stanzspalts, in einem Abschnitt der einlagig oder mehrlagig vorliegenden Folie oder Beutels oder das Bereitstellen einer Folie oder eines Beutels mit einer solchen Durchgangsöffnung. Ferner umfasst es das Anordnen des Folienstreifens auf beiden Seiten der Durchgangsöffnung oder das Anlegen des Folienstreifens an die Folie auf beiden Seiten der Durchgangsöffnung sowie das Verbinden, z. B. Verschweißen oder Verkleben oder Stanznieten, des Folienstreifens mit sich selber durch die Durchgangsöffnung hindurch.

Erfindungsgemäß wird oder ist der Folienstreifen nicht mit der Folie verbunden, sondern ausschließlich mit sich selbst.

In manchen erfindungsgemäßen, beispielhaften Ausführungsformen des Produkts weist die Folie oder der Beutel in wenigstens einem Abschnitt, in welchem sie einlagig oder mehrlagig vorliegt, eine Durchgangsöffnung auf. Dabei liegt der Folienstreifen auf beiden Seiten der Durchgangsöffnung vor und ist mit sich selbst durch die Durchgangsöffnung verbunden.

"Einlagig" kann in bestimmten erfindungsgemäßen, beispielhaften Ausführungsformen eine Folienlage bedeuten, wobei die Folienlage genau eine Innenseite und genau eine Außenseite aufweist, wobei die Innenseite Kontakt mit dem medizinischen Produkt haben kann und die Außenseite Kontakt mit der Umgebung oder Atmosphäre haben kann.

"Zweilagig" kann in bestimmten erfindungsgemäßen, beispielhaften Ausführungsformen zwei Folienlagen bedeuten, wobei jede der beiden Folienlagen genau eine Innenseite und genau eine Außenseite aufweist, wobei jede Innenseite Kontakt mit dem medizinischen Produkt haben kann und jede Außenseite Kontakt mit der Umgebung oder Atmosphäre haben kann.

Die Kennzeichnung kann erfindungsgemäß auf jede beliebige Weise erfolgen, vorzugsweise und rein exemplarisch auf eine der folgenden Weisen: Inkjet-, Laser-, Thermotransfer- oder Heißprägeverfahren.

Als Codes können erfindungsgemäß rein beispielhaft StrichCodes, 2-D-Codes, Data-Matrix-Codes oder andere verwendet werden.

Die Beutelfolien sind in gewissen erfindungsgemäßen Ausführungsformen transparent. In diesen Ausführungsformen ist lediglich der Folienstreifen farbig oder schwarz gestaltet. Vorteilhaft bei der farbigen oder schwarzen Gestaltung ist die bessere Lesbarkeit durch Erhöhung des Kontrasts zwischen Untergrund und Aufschrift (z. B. schwarzer Code auf weißem Hintergrund), so dass auch eine bessere automatische Einlesbarkeit gewährleistet ist.

Manche oder alle erfindungsgemäßen Ausführungsformen können einen, mehrere oder alle der oben und/oder im Folgenden genannten Vorteile aufweisen.

Ein möglicher Vorteil des erfindungsgemäßen Verfahrens zum Kennzeichnen einer Folie oder eines Beutels mit Angaben zum hierin enthaltenen medizinischen Produkt kann darin bestehen, dass die erfindungsgemäße Kennzeichnung in Form von Codes, Klarschrift oder Symbolen im Rahmen einer Serialisierung von Produkten eine Erzeugung von variablen Daten und damit eine bessere Nachvollziehbarkeit bei Fehlern und Reklamationen ermöglicht, da man anstelle ganzer Chargen Einzelprodukte zurückverfolgen kann. Auch ist eine detaillierte Prozessverfolgung z. B. im Zusammenhang mit Herstellungsberichten vorteilhaft möglich.

Mithilfe des erfindungsgemäßen Verfahrens können erfindungsgemäß erstmals auch solche Folienmaterialien vorteilhaft als Folienstreifen verwendet werden, die besonders gut geeignete Oberflächeneigenschaften für eine gute Farbhaftung und/oder eine gute Farbdarstellung für die Kennzeichnung aufweisen, ohne diese Folien auch als Folie für die vollständige Ummantelung des medizinischen Produkts einsetzen zu müssen.

Der Folienstreifen mit der Kennzeichnung kann vorzugsweise derart mit der Folie verbunden sein, dass der Folienstreifen - vorzugsweise jedenfalls im Gebrauch oder bei der üblichen Handhabung in der Anwendungsumgebung, d. h. beispielsweise außerhalb eines Labors oder dergleichen - nur zerstörend von der Folie des Beutels gelöst oder getrennt werden kann. Dies ist vorteilhaft, da die Markierung oder Kennzeichnung des medizinischen Produkts fest mit dem medizinischen Produkt selbst bzw. dessen Folie oder Beutel verbunden sein und vor allem bleiben soll.

Weiterhin vorteilhaft kann die Farbe der Folie des Beutels für das medizinische Produkt unabhängig von der Farbe der Kennzeichnung ausgewählt werden. Beispielsweise könnte die Folie weiß sein, und die Kennzeichnung oder einzelne Bestandteile der Kennzeichnung könnte bei geeigneter Wahl einer weiteren Farbe (außer weiß) für den Untergrund der Kennzeichnung ebenfalls weiß sein.

Aufgrund der getrennten Ausführung der Folie einerseits und des Folienabschnitts mit der Kennzeichnung andererseits kann die Markierung vorteilhaft einfach ausgelesen und detektiert werden. Ein erforderlicher Kontrast kann frei gewählt werden. Dies ist insbesondere dann vorteilhaft, wenn eine bestimmte Güte oder Qualität der Markierung beispielsweise bei der Zulassung medizinischer Produkte gefordert wird, wie z. B. ein bestimmter oder ausreichender Kontrast (etwa schwarz/weiß Kontrast).

Weiterhin kann mittels des erfindungsgemäßen Verfahrens vorteilhaft erreicht werden, dass bei einer Markierung oder Kennzeichnung mittels eines Laserverfahrens (z. B. bei Laserfarbtransferkennzeichnungen) die Folie des Beutels intakt bleibt und ihre Barriereneigenschaft nicht beeinträchtigt wird, auch wenn die Laserparameter oder -auswirkung einer Schwankung unterliegen.

Das Versehen eines Folienabschnitts mit der Kennzeichnung unabhängig von Ort und Zeitpunkt der Herstellung und ggf. auch Befüllung beispielsweise eines Beutels mit einem medizinischen Produkt und des Verbindens von Folienabschnitt mit Folie ermöglicht weiterhin vorteilhaft, dass der bedruckte Folienabschnitt unabhängig von einer Taktung einer Produktionslinie zur Herstellung der befüllten Beutel erfolgen kann. Die Zusammenführung des bedruckten Folienabschnitts mit dem befüllten Beutel wird lediglich durch den Fügevorgang oder Verbindevorgang, z. B, durch den Schweißvorgang, selbst vollzogen.

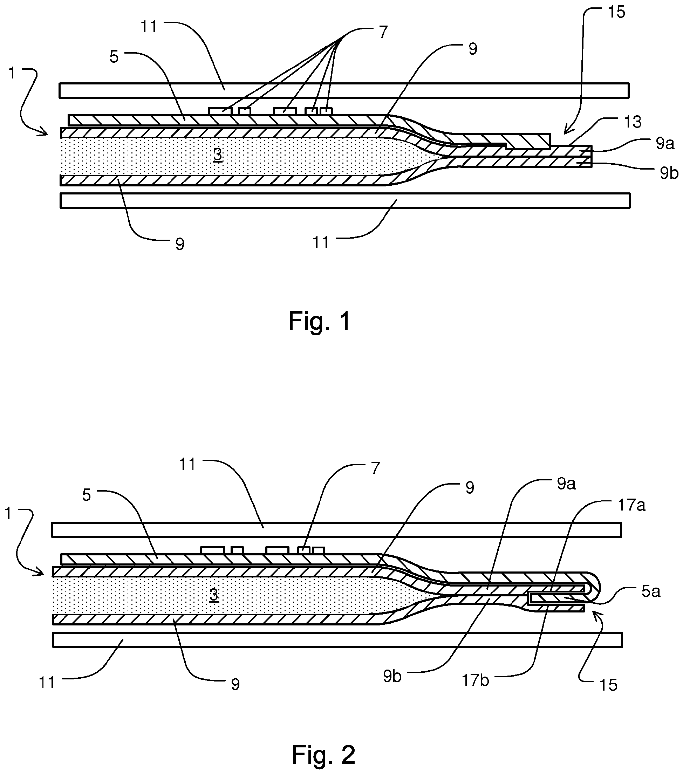

Eine direkte Verbindung eines bedruckten Folienabschnitts mit der Oberfläche einer Folie eines Beutels kann in bestimmten Ausführungsformen vorteilhaft vermieden werden, indem der bedruckte Folienabschnitt mittels einer innenliegenden Verbindung wie einer Verklebung, Verschweißung oder dergleichen mit der Folie verbunden wird (siehe Fig. 2). Dadurch können vorteilhaft Oberflächen, Außenflächen oder Außenschichten für die Folie verwendet werden, die einerseits schlecht oder überhaupt nicht verbindbar sind, andererseits aber gute anderweitige Materialeigenschaften (z. B. hohe Barriereneigenschaften wie etwa SiOₓ-Folienbeschichtungen) aufweisen.

Weiterhin ermöglicht das erfindungsgemäße Verfahren in bestimmten Ausführungsformen vorteilhaft, Materialien für den bedruckbaren Folienabschnitt auszuwählen und zu verwenden, die nicht mit der Folie für den Beutel verbindbar oder verschweißbar sind, sondern nur mit sich selbst. Damit kann vorteilhaft das Material für den Folienabschnitt einerseits und für die Folie des Beutels andererseits unabhängig voneinander ausgewählt werden kann.

Mittels des erfindungsgemäßen Verfahrens kann es schließlich vorteilhaft möglich sein, die Fälschungsrichtlinie 2011/62/EU für medizinische Produkte in Beuteln durch eine beutelspezifische individuelle Kennzeichnungspflicht (Serialisierung) anzuwenden. Das erfindungsgemäße Verfahren ermöglicht es ferner, in einem ersten Schritt variable Daten zur Durchführung der Kennzeichnung zu generieren und in einem zweiten Schritt diese Daten als individuelle Kennzeichnung fest mit dem Beutel, der das medizinische Produkt beinhaltet, zu verbinden.

Alle mit dem erfindungsgemäßen Verfahren erzielbaren Vorteile lassen sich in bestimmten erfindungsgemäßen Ausführungsformen ungeschmälert auch mit dem erfindungsgemäßen Produkt erzielen.

Im Folgenden wird die vorliegende Erfindung unter Bezugnahme auf die beigefügten Figuren rein exemplarisch beschrieben. Es gilt:
- Fig. 1: zeigt in einer vereinfachten Schnittdarstellung ein medizinisches Produkt in einem (Folien-) Beutel mit einer Folie, welcher einen Folienstreifen mit Angaben zur Kennzeichnung des medizinischen Produkts aufweist, in einer nicht erfindungsgemäßen Ausführungsform;
- Fig. 2: zeigt den Beutel aus Fig. 1 mit einem Folienstreifen mit Angaben zur Kennzeichnung des medizinischen Produkts, in einer nicht erfindungsgemäßen Ausführungsform;
- Fig. 3: zeigt den Beutel aus Fig. 1 mit einem Folienstreifen mit Angaben zur Kennzeichnung des medizinischen Produkts, in einer erfindungsgemäßen Ausführungsform; und die
- Fig. 4 bis 6: zeigen jeweils Schritte zum Aufbringen der Kennzeichnung auf den Folienstreifen sowie die anschließende Verbindung des Folienstreifens mit dem Beutel.

Die folgenden Figuren beschreiben die vorliegende Erfindung in einem Beispiel, in welchem ein Folienstreifen mit einer Folie verschweißt wird. Dieses Beispiel ist rein exemplarisch. Andere Verbindungsverfahren und Fügeverfahren, bei welchen nicht verschweißt oder nicht ausschließlich verschweißt wird, sind ebenfalls von der vorliegenden Erfindung umfasst, werden aber zur Vermeidung von Wiederholungen nicht eigens mittels Figuren erläutert.

**Fig. 1** zeigt in einer vereinfachten Schnittdarstellung einen Abschnitt eines Beutels 1, welcher ein medizinisches Produkt 3 (hier ein medizinisches Verbrauchsmaterial wie etwa Dialysierflüssigkeit oder -lösung) umgibt, sowie einen Folienstreifen 5 mit Angaben zur Kennzeichnung 7 des medizinischen Produkts 3. Der Beutel 1 ist aus einer Folie 9 hergestellt, entspricht dieser in dieser nicht erfindungsgemäßen Ausführungsform, oder weist zumindest eine Folie 9 auf.

Der Beutel 1 ist beispielhaft mit einer äußeren Folie 11 umschlossen, verpackt oder geschützt, welche entsprechend als Umfolie, Schutzfolie oder Verpackung bezeichnet werden kann. Die äußere Folie 11 ist in den Figuren nur schematisch angedeutet. Sie ist optional, und falls vorhanden, vorzugsweise den Beutel 1 vollständig umgebend vorgesehen.

Die Kennzeichnung 7 kann auf unterschiedliche Art und Weise auf den Folienstreifen 5 aufgebracht werden. Beispielsweise kann der Folienstreifen 5 mittels einer Tinte bedruckt werden. Ergänzend oder alternativ kann der Folienstreifen 5 mittels eines Laserverfahrens oder Beklebung gekennzeichnet werden.

In der rein exemplarischen nicht erfindungsgemäßen Ausführungsform der Fig. 1 ist der Folienstreifen 5 mittels wenigstens einer Verschweißung oder Schweißstelle 15 mit einer Außenfläche 13 (oder einer Außenschicht oder ersten Folienlage 9a, wenn die Folie 9 wie im Beispiel der Fig. 1 mehrlagig ausgestaltet ist) der Folie 9 verbunden. Hierzu sind das Material des Folienstreifens 5 und das Material der Folie 9 ausgewählt, um miteinander verschweißbar zu sein. In anderen nicht erfindungsgemäßen, beispielhaften Ausführungsformen, in welche der Folienstreifen 5 nicht mittels Schweißens mit Folie 9 verbunden wird sondern etwa durch Verkleben, ist die Verschweißung oder Schweißstelle 15 folglich eine Verklebung oder Verklebestelle, usw.

Für diesen Zweck kommen insbesondere Thermoplaste als Folienmaterial und Folienstreifenmaterial in Frage, da diese in der Regel durch Wärmeeinwirkung erweichen, plastisch verformbar fließfähig werden und schweißbar sind. In Betracht kommen beispielsweise Thermoplasten wie Polypropylen (PP) oder Polyethylen (PE), welche mit den üblichen, insbesondere den hierin genannten Fügeverfahren verbindbar, z. B. verschweißbar sind, ebenso Polyethylenterephthalat (PET).

Die Fläche der Verschweißung 15 kann beispielsweise streifenförmig, nahtförmig oder lappenförmig (die Längsausrichtung der Streifen/der Naht oder des Lappens verläuft in Fig. 1 senkrecht zur Zeichenebene) ausgebildet sein.

In dem in Fig. 1 rechts gezeigten Ende des Beutels 1 liegt die Folie 9 in einer ersten Folienlage 9a einer zweiten Folienlage 9b an und ist dort mit ersterer zumindest abschnittsweise verschweißt. Diese Verschweißung ist in Fig. 1 nicht dargestellt, und sie ist unabhängig von der Schweißstelle 15, in welcher der Folienstreifen 5 mittels Schweißens mit der Folie 9 verbunden ist.

Die Verschweißung 15 ist im Beispiel der Fig. 1 insbesondere am äußeren Rand des Beutels 1 oder im Bereich, in welchem sich die erste Folienlage 9a und die zweite Folienlage 9b überlappen und ihrerseits miteinander verschweißt sind, erfolgt. Der Bereich, in welchem die erste Folienlage 9a und die zweite Folienlage 9b miteinander verschweißt sind, kann bezogen auf die Verschweißung 15 weiter innen (zum Beutelinhalt bzw. zum medizinischen Produkt 3 hin gerichtet) angeordnet sein, sich mit dem Bereich der Verschweißung 15 des Folienstreifens 5 mit der Folie 9 überlappen oder weiter außen (in Fig. 1 nach rechts) angeordnet sein.

**Fig. 2** zeigt den Beutel 1 der Fig. 1, jedoch ist der Folienstreifen 5, welche die Angaben zur Kennzeichnung 7 des medizinischen Produkts 3 trägt, auf andere Weise an der Folie 9 befestigt als in Fig. 1

Im Gegensatz zur Darstellung der Fig. 1 ist der Folienstreifen 5 in der zweiten Ausführungsform der Fig. 2 mit wenigstens einem Abschnitt 5a hiervon zwischen einem Abschnitt der ersten Folienlage 9a und einem Abschnitt der zweiten Folienlage 9b angeordnet.

Der Folienstreifen 9 ist im Beispiel der Fig. 2 dabei mit zwei Innenflächen der Folie 9 bzw. mit jeweils der Innenfläche 17a der ersten Folienlage 9a und der Innenfläche 17b der zweiten Folienlage 9b verschweißt. Insbesondere ist der Folienstreifen 9 im Bereich der Verschweißung 15 beidseitig, d. h. mit seinen beiden Außenflächen (in Fig. 2 also mit Ober- und Unterseite), mit jeweils den beiden im Beispiel der Fig. 2 einander zugewandten Innenflächen 17a der ersten Folienlage 9a bzw. der Innenfläche 17b der zweiten Folienlage 9b der Folie 9 verschweißt. In anderen nicht erfindungsgemäßen Ausführungsformen ist der Folienstreifen mit seinem Abschnitt 5a mit nur einer der Innenflächen 17a, 17b verschweißt.

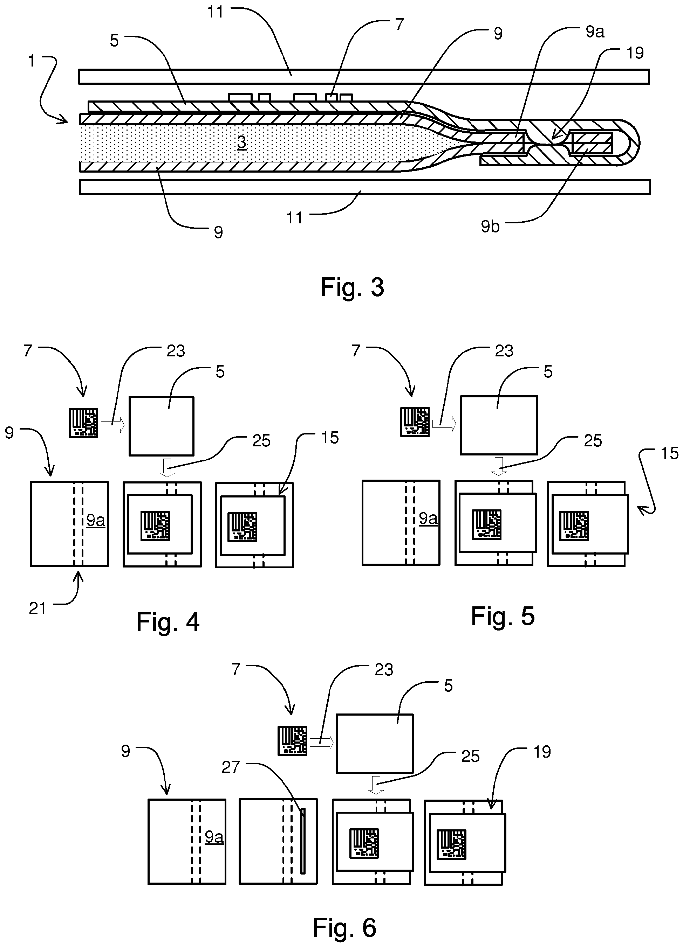

**Fig. 3** zeigt den Beutel 1 aus Fig. 1 mit dem medizinischen Produkt 3 sowie eine weitere Anordnung und Befestigung des Folienstreifens 5 an der Folie 9 bzw. dem Beutel 1.

Im Gegensatz sowohl zur Fig. 1 als auch zur Fig. 2 ist in Fig. 3 der Folienstreifen 5 nicht mit der Folie 9 des Beutels 1 verschweißt, sehr wohl aber mit sich selbst, und zwar in wenigstens einer Verschweißung 19. Konkret ist in der Verschweißung 19 ein Abschnitt des Folienstreifens 5 mit einem weiteren Bereich des Folienstreifens 5 verschweißt, und vorzugsweise ausschließlich mit sich selbst. Optimalerweise ist das Material des Folienstreifens 5 hierzu mit sich selbst verschweißbar. Alternativ könnte der Folienstreifen 5, wenigstens im Bereich der Verschweißung 19 des Folienstreifens 5 mit sich selbst geeignet beschichtet sein oder ein anderes oder modifiziertes Material aufweisen, um die Verschweißbarkeit des Folienstreifen bzw. seiner Beschichtung mit sich selbst zu gewährleisten.

Die Verschweißung des Folienstreifens 5 mit sich selbst ist im Beispiel der Fig. 3 möglich, da der Beutel 1 in seinem (in Fig. 3) rechten Endbereich, in welchem die erste Folienlage 9a und die zweite Folienlage 9b miteinander verbunden sind, eine Durchgangsöffnung aufweist, welche durch Oberfläche wie Unterfläche des Beutels 1 hindurch reicht. Die Durchgangsöffnung kann ein durchgängiger Schlitz 27 sein, wie er in Fig. 6 gezeigt und zu Fig. 6 erläutert ist. Andere Formen als die Schlitzform, etwa rund, rechteckig oder dergleichen sind für die Durchgangsöffnung ebenfalls möglich und ebenfalls von der vorliegenden Erfindung umfasst.

Aufgrund der Durchgangsöffnung kann eine Verschweißung des Folienstreifens mit sich selbst durch die Folie 9 hindurch erfolgen. Die Verschweißung des Folienstreifens 5 durch die Durchgangsöffnung hindurch legt den Folienstreifen 5 formschlüssig bezogen auf die Folie 9 oder den Beutel 1 fest, ohne hiermit stoffschlüssig oder kraftschlüssig verbunden sein zu müssen. Dies erlaubt es vorteilhaft, Materialien für die Folie 9 einerseits und für den Folienstreifen 5 andererseits auszuwählen, welche nicht oder möglicherweise nur mit besonderem Aufwand miteinander verschweißbar sind.

**Fig. 4** zeigt mögliche Schritte, und was gewisse Ausführungsformen betrifft sogar alle Schritte, die zum Aufbringen der Kennzeichnung 7 auf den Folienstreifen 5 sowie zum anschließenden Verbinden des Folienstreifens 5 mit der Folie 9 des Beutels 1 entsprechend der Ausführungsform der Fig. 1 erforderlich sind.

Der Bereich einer Verschweißung oder Schweißnaht 21 der sich überlappenden ersten und zweiten Folienlagen 9a, 9b der Folie 9 (also die Verschweißung der Folienlagen 9a, 9b zum Abdichten des Beutels 1) ist strichpunktiert als Streifen dargestellt.

Der Bereich der Verschweißung 15 des Folienstreifens 5 mit der Folie 9 oder der ersten Folienlage 9a liegt insbesondere im äußeren Bereich (in Fig. 4 im rechten Endbereich der Folie 9).

Zunächst wird in einem ersten Schritt 23 die Kennzeichnung 7 auf den Folienstreifen 5 aufgebracht. Dies kann beispielsweise durch Bedrucken des Folienstreifens 5 mit Tinte oder mit Hilfe eines Laserbeschriftungsverfahrens erfolgen. Anschließend wird in einem zweiten Schritt 25 der bedruckte oder anderweitig gekennzeichnete Folienstreifen 5 mit der Folie 9 verschweißt. Der Folienstreifen 5 überdeckt dabei vorzugsweise nicht die gesamte Breite der Folie 9.

Es wird angemerkt, dass die Schritte 23 und 25 erfindungsgemäß auch in umgekehrter Reihenfolge als vorstehend beschrieben erfolgen könnten. Dies gilt auch für die Ausführungsformen, die zu den Fig. 5 und 6 beschrieben sind.

**Fig. 5** zeigt analog zur Fig. 4 mögliche Schritte, und optional sogar alle Schritte, zum Aufbringen der Kennzeichnung 7 auf dem Folienstreifen 5 sowie zum anschließenden Verschweißen des Folienstreifens 5 mit der Folie 9 des Beutels 1 entsprechend der Ausführungsform der Fig. 2.

Im Bereich der Verschweißung 15 des Folienstreifens 5 ist dieser mit seinem Abschnitt 5a mit den Innenflächen 17 (siehe Fig. 2) der Folie 9 verbunden, was in Fig. 5 nicht erkennbar ist.

Anders als in Fig. 4 ragt der Folienstreifen 5 deshalb über den rechten Rand der Folie 9, jedenfalls aber über den Rand einer Folienlage (hier der ersten Folienlage 9a) über.

**Fig. 6** zeigt analog zur Fig. 4 mögliche Schritte, und optional sogar alle Schritte, zum Aufbringen der Kennzeichnung 7 auf den Folienstreifen 5 sowie zum anschließenden Verbinden des Folienstreifens 5 mit der Folie 9 des Beutels 1 entsprechend der Ausführungsform der Fig. 3.

Im Gegensatz zu Fig. 4 und Fig. 5 ist der Folienstreifen 5 in Fig. 6 nicht mit der Folie 9 vorzugsweise nicht stoffschlüssig und optional auch nicht kraftschlüssig verbunden, insbesondere aber nicht verschweißt. Vielmehr ist ein Abschnitt des Folienstreifens 5 mit einem weiteren Abschnitt des Folienstreifens 5, also mit sich selbst, verschweißt.

Zum Zusammenführen dieser beiden zu verschweißenden Abschnitte des Folienstreifens 5 weist die Folie 9 beispielhaft eine Durchgangsöffnung, vorzugsweise, da einfach herzustellen, einen durchgängigen Schlitz 27 auf.

Die Durchgangsöffnung, hier also der Schlitz 27, weist vorzugsweise eine Länge auf, welche mindestens so groß ist wie die Breite des Folienstreifens 5. Auf diese Weise lässt sich der Folienstreifen 5 ohne Kraftaufwand und ohne sich zu verwerfen, was sein Verschweißen erschweren könnte, durch die Durchgangsöffnung führen.

In der rechten Abbildung der Fig. 6 ist dieser durchgängige Schlitz 27 im Bereich der Verschweißung von dem die Kennzeichnung 7 tragenden Folienstreifen 5 überdeckt und nicht sichtbar.

Der Folienstreifen 5 kann einstückig sein. Er kann aber auch zwei- oder mehrteilig gefertigt sein. Ist er mehrteilig gefertigt, so wird ein erster Abschnitt hiervon auf der Oberseite der Folie 9, also auf der ersten Folienlage 9a, platziert, ein zweiter Abschnitt hiervon auf der Unterseite der Folie 9, also auf der zweiten Folienlage 9b. Ein Verschweißen erfolgt durch die Durchgangsöffnung hindurch wie vorstehend beschrieben. Sind die beiden Abschnitte groß genug gefertigt, so ist der Folienstreifen 5 insgesamt formschlüssig in der Durchgangsöffnung bzw. der Folie 9 festgelegt.

### Bezugszeichenliste

- 1: Beutel
- 3: medizinisches Produkt
- 5: Folienstreifen
- 5a: Abschnitt des Folienstreifens
- 7: Kennzeichnung; Angaben zur Kennzeichnung
- 9: Folie
- 9a: erste Folienlage
- 9b: zweite Folienlage
- 11: äußere Folie
- 13: Außenfläche, Außenschicht der Folie
- 15: Verschweißung des Folienstreifens mit der Folie
- 17a: Innenfläche, Innenschicht der ersten Folienlage 9a
- 17b: Innenfläche, Innenschicht der zweiten Folienlage 9b
- 19: Verschweißung des Folienstreifens mit sich selbst Schweißnaht oder Verschweißung der Folien zum
- 21: Abdichten des Beutels Schritt zum Aufbringen einer Kennzeichnung auf den
- 23: Folienstreifen Schritt zum Aufbringen des gekennzeichneten
- 25: Folienstreifens auf die Folie
- 27: durchgängiger Schlitz

## Patentansprüche

1. Verfahren zum Kennzeichnen einer Folie (9) oder eines zumindest abschnittsweise eine Folie (9) aufweisenden Beutels (1), wobei die Folie (9) oder der Beutel (1) ein medizinisches Produkt (3) aufweist oder umgibt oder für dessen Aufnahme vorgesehen oder vorbereitet ist, wobei das Verfahren den Schritt umfasst:
- Verbinden eines Folienstreifens (5) durch ein Fügeverfahren an der Folie (9), wobei der Folienstreifen (5) Angaben zur Kennzeichnung des medizinischen Produkts (3) trägt, wobei der Folienstreifen (5) nicht mit der Folie (9) verbunden wird, sondern ausschließlich mit sich selbst verbunden wird,
**dadurch gekennzeichnet, dass**
der Folienstreifen (5) durch Verschweißen mit sich selbst verbunden wird,
wobei der Folienstreifen (5) aus einem Thermoplast besteht.

2. Verfahren nach einem der vorangegangenen Ansprüche, mit dem Schritt:
- Erzeugen einer Durchgangsöffnung (27) in einem Abschnitt der Folie (9);
- Anordnen des Folienstreifens (5) auf beiden Seiten der Durchgangsöffnung (27); und
- Verbinden des Folienstreifens (5) mit sich selbst durch die Durchgangsöffnung (27) hindurch.

3. Beutel und medizinisches Produkt (3), wobei der Beutel zumindest abschnittsweise aus Folie (9) besteht, wobei das medizinische Produkt innerhalb oder unter der Folie (9) vorliegt, wobei wenigstens ein Folienstreifen (5) mit dem Beutel (1) an der Folie (9) durch ein Fügeverfahren verbunden oder befestigt ist, wobei der Folienstreifen (5) Angaben zur Kennzeichnung des medizinischen Produkts (3) trägt, wobei der Folienstreifen (5) nicht mit der Folie (9) verbunden ist, sondern ausschließlich mit sich selbst,
**dadurch gekennzeichnet, dass**
das verwendete Fügeverfahren ein Verschweißen ist,
wobei der Folienstreifen (5) aus einem Thermoplast besteht.

4. Produkt nach Anspruch 3, wobei die Folie (9) in wenigstens einem Abschnitt, in welchem sie einlagig oder mehrlagig vorliegt, wenigstens eine Durchgangsöffnung (27) aufweist; wobei der Folienstreifen (5) auf beiden Seiten der Durchgangsöffnung (27) vorliegt; und wobei der Folienstreifen (5) mit sich selbst durch die Durchgangsöffnung (27) hindurch verbunden ist.

## Claims

1. A method for marking a film (9) or a bag (1) comprising a film (9) at least in portions, wherein the film (9) or the bag (1) comprises or surrounds a medical product (3), or is provided or prepared for its reception, the method comprising the step of:
- connecting a film strip (5) by means of a joining process on the film (9), wherein the film strip (5) carries indications marking the medical product (3), the film strip (5) not being connected to the film (9) but exclusively to itself,
**characterized in that**
the film strip (5) is connected to itself by welding,
the film strip (5) consists of a thermoplastic.

2. The method according to anyone of the preceding claims comprising the step of:
- creating a through-opening (27) in a portion of the film (9);
- arranging the film strip (5) on both sides of the through-opening (27); and
- connecting the film strip (5) with itself via the through-opening (27).

3. A bag and a medical product (3),
wherein the bag is a film (9) at least in portions, wherein the medical product is present inside or below the film (9),
wherein at least one film strip (5) is connected with or attached to the bag (1) on the film (9) by a joining process, wherein the film strip (5) carries indications marking the medical product (3), the film strip (5) not being connected to the film (9) but exclusively to itself,
**characterized in that**
the joining process used is welding,
the film strip (5) consists of a thermoplastic.

4. A product according to claim 3, wherein the film (9) comprises in at least one portion in which it is single or multi-layered at least one through-opening (27);
the film strip (5) being present on both sides of the through-opening (27) and the film strip (5) being connected to itself via the through-opening (27).

## Revendications

1. Un procédé de caractérisation d'un film (9) ou d'un sachet (1) présentant au moins par sections un film (9),
où le film (9) ou le sachet (1) comprend, ou entoure, un produit médical (3), ou est prévu, ou préparé, pour sa réception, le procédé comprenant l'étape consistant à:
- relier une bande de film (5) au moyen d'un processus d'assemblage sur le film (9), où la bande de film (5) porte des indications destinées à la caractérisation du produit médical (3), la bande de film (5) n'étant pas reliée au film (9) mais exclusivement à elle-même,
**caractérisé en ce que**
la bande de film (5) est reliée à elle-même par soudage,
la bande de film (5) est constituée d'un thermoplastique.

2. Le procédé selon l'une quelconque des revendications précédentes, comprenant l'étape consistant à:
- créer une ouverture de passage (27) dans une section du film (9);
- agencer la bande de film (5) des deux côtés de l'ouverture de passage (27); et
- relier la bande de film (5) à elle-même via l'ouverture de passage (27).

3. Un sachet et un produit médical (3),
où le sachet est constitué au moins par sections d'un film (9), où le produit médical est présent à l'intérieur ou sous le film (9),
où au moins une bande de film (5) est reliée ou attachée au sachet (1) sur le film (9) via un processus d'assemblage, où la bande de film (5) porte des indications destinées à la caractérisation du produit médical (3), la bande de film (5) n'étant pas reliée au film (9) mais exclusivement à elle-même,
**caractérisé en ce que**
le processus d'assemblage utilisé est le soudage,
la bande de film (5) est constituée d'un thermoplastique.

4. Un produit selon la revendication 3, où le film (9) comporte dans au moins une section dans laquelle il est monocouche ou multicouche au moins une ouverture de passage (27);
la bande de film (5) étant présente des deux côtés de l'ouverture de passage (27) et la bande de film (5) étant reliée à elle-même via l'ouverture de passage (27).
